# EUROPEAN PATENT SPECIFICATION

(11) **EP 1 510 225 B1**
(45) Date of publication and mention of the grant of the patent: **14.05.2014**
(21) Application number: 04028866.4
(22) Date of filing: 03.06.1996
(51) Int. Cl.: A61K 31/197, A61K 41/00, A61K 49/00, A61P 17/10, A61K 9/08

(54) **Photochemotherapeutic use of 5-ALA in the treatment of acne**
5-ALA als Photochemotherapeuticum zur Behandlung von Akne
5-ALA en application photochémotherapeutique dans le traitement de l'acné

(30) Priority: 05.06.1995 US 465242
(43) Date of publication of application: 02.03.2005
(62) Divisional of application: 96915923.5
(73) Proprietor: QUEEN'S UNIVERSITY AT KINGSTON, Kingston, Ontario K7L 3N6 (CA)
(72) Inventor: Kennedy, James C., Kingston Ontario Canada K7M 4A6 (CA); Pottier, Roy H., Kingston Ontario Canada K7L 4V1 (CA); Reid, Robert L., Kingston Ontario Canada K7L 4V1 (CA); Sac-Morales, Arnold, Kingston Ontario Canada K7K 6G8 (CA); Tomalty, Lewis L., Kingston, Ontario/ K7L 2M4 (CA)
(74) Representative: Murphy, Colm Damien

(56) References cited:
- WO-A-91/01727
- WO-A-93/20810
- WO-A-94/06424
- WO-A-95/05813
- WO-A-95/07077
- WO-A-95/31189
- DATABASE EMBASE ELSEVIER SCIENCE PUBLISHERS, AMSTERDAM, NL; AN=96020997 XP002009953 & SAMSOEN M.: "ARSENAL MEDICAMENTEUX DERMATOLOGIQUE 1995" NOUVELLES DERMATOLOGIQUES, vol. 14, no. 10, 1995, pages 603-606, FRANCE
- HONGCHARU ET AL: "Topical ALA - photodynamic therapy for the treatment of acne vulgaris", JOURNAL OF INVESTIGATIVE DERMATOLOGY, NATURE PUBLISHING GROUP, GB, vol. 115, no. 2, 1 January 2000 (2000-01-01), pages 183-192, XP002305494, ISSN: 0022-202X, DOI: 10.1046/J.1523-1747.2000.00046.X
- LOZOVAYA G I ET AL: "PROTOPORPHYRIN IX AS A POSSIBLE ANCIENT PHOTOSENSITIZER SPECTRAL AND PHOTOCHEMICAL STUDIES", ORIGINS OF LIFE AND EVOLUTION OF THE BIOSPHERE, vol. 20, no. 3-4, 1990, pages 321-330, & 1989 ISSOL (INTERNATIONAL SOCIETY FOR THE ORIGIN OF LIFE) MEETING, PART II, PRAGUE, CZECHOSLOVAKIA. ISSN: 0169-6149

## Description

### Field of Invention

This invention relates to the detection and treatment, by induced fluorescence and photochemotherapy, respectively, of certain tissue abnormalities (both cancerous and non-malignant of endogenous and exogenous origin), hyperproliferative cells, and normal cells. The invention also relates to the detection and treatment of abnormalities in body fluids or suspensions of tissues containing abnormal cells by induced fluorescence and photochemotherapy.

### Background of Invention

Tissue abnormalities involving the skin usually are detected and assessed by a combination of visual inspection and palpation. In certain clinical situations the sensitivity of the visual inspection can be enhanced by the use of non-white light (either ultraviolet or a narrow band in the visible), or by the prior application of a contrast-enhancing agent such as dilute acetic acid or certain stains. Tissues abnormalities that involve surfaces that cannot be palpated (such as the bronchi or the urinary bladder) may be visualized via an appropriate scope. Some specialized scopes can detect induced fluorescence. If the abnormality in question is associated with a difference in either the extent or the pattern of tissue vascularization, such a scope may be used to determine the limits of the area involved by the abnormality, by visualizing an injected bolus of fluorescein or other fluorescent material as it passes through the vasculature of both the lesion and the adjacent normal tissue.

In addition, fluorescence-detecting scopes are being used experimentally to identify areas of tissue that show strong porphyrin fluorescence following the intravenous injection of exogenous porphyrins such as hematophorphyrin IX (HpIX), hematoporphyrin derivative (HpD), or "dihematoporphyrin ether". Such porphyrins tend to accumulate semi-preferentially in malignant tissues, but they also accumulate in tissues that are regenerating following an injury or in the rapidly growing tissues of an embryo or fetus. Normal liver, spleen, and kidney also tend to accumulate these porphyrins. Using such compounds and fluorescence-detecting scopes, areas of malignant tissue too small to be identified by standard forms of visual inspection have been identified in the bronchi and in the urinary bladder.

Unfortunately, a clinically significant (photosensitizing) amount of porphyrin may persist in the skin for at least two weeks, (occasionally for more than two months) following the intravenous injection of HpIX, HpD, or a semi-puridied preparation of HpD, such as Photofrin II. (Photophrin is a registered trademark of Quadra Logics, Inc. Vancouver, British Columbia, Canada.) This means that patients must avoid exposure to sunlight (either direct, or through window glass) for an inconveniently long period of time post-injection. Understandably, patient compliance often is poor, and accidental phototoxic "sunburn" is a common occurrence in the weeks following a diagnostic or therapeutic injection of porphyrin. Persistent photosensitivity is the major hazard associated with this technique, and is the main reason why it is not used more widely.

The standard treatments for cancer comprise surgery, radiotherapy and chemotherapy. However, other forms of treatment are also known, including photochemotherapy or photodynamic therapy (PDT), based on the discovery made over 90 years ago that unicellular organisms, .*i.e*., certain rapidly growing cells (such as cells of the Lower Kingdom, now referred to as *Protista*), treated with certain chemicals will die when exposed to light. Thus, synthetic porphyrins have been shown *in vitro* to protect cells from infections such as parasites, *e.g*., tyromastigotes and sphaeromastigotes of Tyropanosoma cruzi, J. Parasitol., 75(6) 1989, p. 970-976, and gram positive bacteria, mycoplasma and yeasts, Malik et al. J. Photochemistry and Photobiology, B. Biology 5 281-293 (1990). *P. acne* is known to, *in vitro*, produce intracellular protoporphyrin in the presence of exogenous ALA. Kjeldstad, Conference on Photosensitization and Photochemotherapy of Cancer, Det Norske Videnskaps-Akademi, March 16-17, 1993, Oslo, Norway.

PDT is currently being used, on an experimental basis, to treat several different types of cancer as well as certain non-malignant lesions such as psoriasis. The patient is given a photo-activatable drug that has some degree of specificity for the tissue being treated. A tissue volume that includes the target tissue is then exposed to photoactivating light so as to destroy the target tissue while causing only mild and reversible damage to the other tissues in the same treatment volume.

There are two main types of photochemotherapeutic agents in clinical use at present. The first type, methoxypsoralens, are given systemically. Ultraviolet light is essential to activate them. Localized exposure of psoralen-containing tissues to ultraviolet light induces a localized photochemical reaction that causes the drug to bind covalently to the DNA of living cells, thus destroying their proliferative potential. The second type, porphyrins and related photosensitizers, are also given systemically (by intravenous injection), although occasionally they are given either topically or by intralesional injection. They can be activated by visible (red) light. The localized exposure of porphyrin-containing tissues to such light ordinarily does not induce a chemical reaction between cell components and the porphyrin molecules. Instead, the porphyrins act as catalysts by trapping the energy of the photoactivating light and then passing it on to molecules of oxygen, which in turn are raised to an excited state that is capable of oxidizing adjacent molecules or structures. Cell death is not caused primarily by damage to the DNA, but by damage to essential membrane structures. The goal of photochemotherapy is sometimes cure (mainly for basal cell carcinomas), but usually the goal is palliation through local control when none of the standard forms of therapy are considered likely to offer a significant degree of benefit to the patient.

Methoxypsoralen (PUVA) therapy is used mainly for the treatment of psoriasis, but sometimes it is also used to treat very superficial cancers that involve the skin (mainly mycosis fungoides). However, there are two serious problems with such treatments. First, the procedure has been demonstrated in humans to be carcinogenic. Second, the depth at which malignant tissue can be killed is limited to a few millimeters below the illuminated surface. These problems severely limit the usefulness of the methoxypsoralens for photochemotherapy.

5-Amino-4-oxopentanoic acid, also known as 5-aminolevulinic acid and as δ-aminolevulinic acid ("ALA") has been described in the cross referenced patents and patent applications first set forth in this specification for detecting and treating rapidly growing cells. ALA has also been reported for use in attenuating the growth and killing plants and insects when applied directly to such organisms followed by exposure to light, based on work of Rebeiz *et al.*

Synthetic porphyrins have also been used as photochemotherapeutic agents in treating rapidly growing, e.g. rapidly dividing or rapidly metabolizing infectious cells, such as infectious pathogens, including protozoal parasites, such as *Plasmodium falciparium* (which causes malaria in humans), various other species of *Plasmodia*, *Leishmania*, and amoebae, pathogenic fungi, and microplasma, including the various parasitic forms, all such cells and organisms being referred to herein as Protista. The term *Protista* as used here and in the literature refers to the lowest orders of the animal and vegetable kingdoms, single celled or collections of single celled organisms including: the eukaryotes, including protozoa, fungi and algae, and the prokaryotes, which are bacteria and blue-green algae.

At present, the porphyrins most commonly used for photochemotherapy are Hematoporphyrin IX (HpIX), Hematoporphyrin derivative (HpD) and various semi-purified preparations of HpD such as commercially available Photofrin^{®} II, a semi-purified form of HpD. When porphyrins are used as photosensitizers, cell death results from damage to cell membranes. Consequently, malignant transformation is not a serious problem. Moreover, since the visible (red) light that is used to photoactivate porphyrins penetrates tissue much more deeply than does the ultraviolet light that must be used to photoactivate methoxypsoralens, the depth at which porphyrin-treated tissue can be killed is substantially greater. Also, since certain types of porphyrins show a significant tendency to accumulate preferentially in malignant tissues, it is sometimes possible to destroy malignant tissue without causing clinically significant damage to adjacent normal tissues.

The main problem with the systemic use of HpIX, HpD and Photofrin II is that photosensitizing concentrations persist in the skin for several weeks to several months following their administration. Consequently, severe accidental phototoxic skin reactions may occur unless the patient avoids exposure to sunlight (either direct, or filtered through window glass) until the concentration of the photosensitizer in the skin has been reduced to a. harmless level. At present, the problem of photosensitivity following the administration of porphyrins is handled by advising the patient to avoid any form of exposure to sunlight (or to very bright artificial lights) for a period of at least two weeks post-injection, and to initiate subsequent exposure to sunlight very cautiously. Not all patients comply with these instructions, since it often is quite inconvenient to do so. In addition, the use of a sunscreen with a high blocking factor is recommended with warning that this will only reduce the hazard somewhat, not eliminate it completely. In a few cases, patients whose photosensitization persisted for more than a month post-treatment have been given large daily doses of beta-carotene over a period of several months in an attempt to prevent accidental phototoxic damage. Finally, attempts have been made to reduce phototoxicity by applying the photosensitizer topically to a limited area.

However, another type of problem is encountered if HpIX or HpD is applied topically in DMSO (dimethylsulfoxide), Azone, or some other vehicle intended to enhance their diffusion through tissue. The porphyrins tend to become immobilized wherever they happened to be when the DMSO or Azone becomes diluted by normal tissue fluids to such an extent that the porphyrins can no longer diffuse through the tissue (or even remain in solution). Consequently, the topical application of porphyrins often is associated with a loss of specificity for malignant tissues, and normal tissues near the site of application may develop persistent photosensitization from the localized concentration of porphyrin.

WO93/20810 published 28 October 1993 discloses medicaments for detecting and treating malignant and non-malignant tissue abnormalities and lesions of the skin; conjunctiva; respiratory, digestive and vaginal mucosa; endometrium and urothelium; and for ablating the endometrial tissue and treating body fluids containing suspended abnormal cells, and for treating cancers of the nervous system with a photochemotherapeutic method using 5-aminolevulinic acid and precursors thereof.

WO95/31189 published 23 November 1995 discloses a medicated wipe comprising an absorbent woven or non-woven fabric, cloth or tissue substrate, impregnated with a pharmaceutically active agent, wherein the agent is a substance effective in stimulating melanocytes to produce melanin and/or is effective in a topical treatment of a skin condition in combination with electromagnetic radiation falling in the range of 220-700nm.

### Obiect of Invention

This invention relates to a method for the detection of certain types of malignant and non-malignant cells including a collection of cells, and tissue abnormalities by induced fluorescence.

It also relates to a photodynamic (photosynthesizing) treatment method using an agent which can be administered either systemically or topically which is not in itself a photosenthesizer but which induces the synthesis or accumulation or both of protoporphyrin IX (PpIX) and other endogenous porphyrins, their precursors and their photoproducts, in rapidly growing cells, including abnormal cells in otherwise normal tissues, in vivo or in vitro.

The terms porphyrin(s) and their precursors refer to compounds produced in vivo in the synthesis of heme and other endogenously produced photoactivatable compounds including their photoproducts.

### Summary of Invention

It is an object of the present invention to provide a precursor of PpIX in the biosynthetic pathway for heme that can induce synthesis and/or accumulation of PpIX or other endogenous porphyrins, their precursors and their photoproducts in non-malignant hyperproliferative lesions, for use in treating of non-malignant hyperproliferative acne lesions of the skin, following exposure of said lesions to light having a wavelength within the photoactivating action spectrum of said PpIX to thereby induce photoactivation in said lesions, wherein the precursor is provided in a solution comprising 20% 5-aminolevulinic acid ("ALA").

This invention is based on the finding that exogenously administered ALA and other precursors of PpIX are metabolized in patients to PpIX and that PpIX preferentially accumulates in rapidly growing cells, as contrasted with less rapidly growing cells. The rapid growth is correlated with the metabolic activity, so that the differential accumulation is affected by the relative metabolic activity between dirrerent cells.

This invention relates to a method for detecting in a patient, a non-malignant lesion or abnormality which is sensitive to PpIX, namely those which preferentially accumulate PpIX, comprising administering to said patient an effective amount of a precursor of PpIX in the biosynthetic pathway for heme so as to induce an accumulation of PpIX in said lesions, and exposing said lesions to light having a wavelength within the absorption spectrum of said PpIX, thereby to induce fluorescence in said lesions.

Another aspect of this invention relates to a method for treating non-malignant hyperproliferative lesions of the skin which are sensitive to PpIX in a patient, comprising administering to said patient an effective amount of a precursor of PpIX in the biosynthetic pathway for heme so as to induce synthesis or accumulation or both of PpIX or other endogenous porphyrins, their precursors and their photoproducts in said lesions, and exposing said lesions to light having a wavelength within the photoactivating action spectrum of said PpIX to thereby induce photoactivation in said lesions.

Thus, the rapidly growing cells involved can be non-malignant hyperproliferative lesions. By another aspect this invention relates to the use of a composition comprising a precursor of protoporphyrin IX in the biosynthetic pathway for heme for the manufacture of a medicament for treating non-malignant tissue abnormalities and lesions.

The preferred precursor of protoporphyrin IX is 5-amino-4-oxo-pentanoic acid, otherwise known as 5-aminolevulinic acid, and a preferred wavelength of the photoactivating light is in the range of 625 to 670 nm, more preferably a red light of 625 to 640 nm.

### Detailed Description of Preferred Embodiment

Protoporphyrin IX (PpIX), a naturally occurring photosensitizer, is the immediate precursor of heme in the heme biosynthetic pathway. All nucleated cells have at least a minimal capacity to synthesize PpIX, since heme is necessary for the synthesis of various essential heme-containing enzymes. Certain types of cells and tissues can synthesize relatively large quantities of PpIX. Under normal conditions, the synthesis of PpIX in such tissues is under such tight feed-back control that the cells produce it at a rate just sufficient to match their need for heme. However, the usual rate-limiting step in the process, the synthesis of 5-aminolevulinic acid, can be bypassed by the provision of exogenous ALA, porphobilinogen, or other precursor of PpIX. Certain tissues and organs will then accumulate such a large excess of PpIX that they become both fluorescent and photosensitive. At least in the case of the skin, the PpIX appears to be synthesized in situ. ALA, which is commercially available from Sigma Chemical Company and other sources and which is water soluble, can be administered orally, topically or by injection. The oral and parenteral routes lead to the induction of clinically useful concentrations of PpIX in certain benign and malignant tissues throughout the body. Only certain types of tissue synthesize and accumulate clinically useful amounts of PpIX when provided with an excess of ALA.

At the present time, treatment of non-malignant hyperproliferative acne lesions of the skin is contemplated.

As used herein the term "skin" includes:
(A) the covering of the external surface of most of the body, commonly termed the skin.
(B) the covering of the external genitalia:
   - labia majora, labia minora, clitoris, and associated structures
   - glans penis, prepuce, and associated structures
(C) the covering of the zone of transition between skin and the mucosa of the digestive system:
   - anal verge
   - vermillion border of the lips
(D) the lining of the external auditory meatus, and the covering of the external surface of the tympanic membrane
(E) all exocrine glands and associated ducts that are located at least partially within an epidermal surface described above, or within the underlying dermis, such as the pilosebaceous units of the skin.

This invention comprises the administration of ALA, other precursors of PpIX and other endogenous porphyrins, to the patient. The administration can also be *in vitro* as applied to tissues of the patient, *i.e., ex vivo*. In *ex vivo* methods, tissue containing the rapidly growing cells are removed from the patient, an effective amount of ALA or endogenous porphyrin is added thereto, then the preparation is subjected to photoactivating light, before being readministered to the patient. The amounts of ALA constituting an effective dose can be determined by one skilled in the art by analogy with the doses used for synthetic porphyrins, based on milligrams per kilogram body weight for *in vivo* systemic application and the typical concentrations for topical or *ex vivo* applications. The compound can be conveniently used orally or intravenously at a dosage of about 10 to 100 mg/kg per single dose, preferedly as a dosage of 40-50 mg/kg; however split dosages of 10 mg/kg four times per day may also be given. The compound can be used topically at a dose of between 2% to 100%, with 100% being dry powder. *Ex vivo* concentrations of the compound are used on cell suspensions in a range of 1-5mM, with a preferred range of 1-2mM; however, if serum is present, a higher dose of about 15 mM should be used. If *ex vivo* use on whole blood, the compound is used at about 15 mM; however, if an iron kelator, such as Desferol™ or des ferroxamine, a lower concentration may be used.

The wavelength of the photoactivating light is of some importance, as it has been shown that between 1 and 10 percent of incident red light (600-700 nm) can pass through a slab of human tissue 1 cm thick, whereas only 0.001 percent or less of blue light (about 400 nm) can pass through the same thickness of human tissue. The photosensitizer will, therefore, be more successful if it absorbs red light. PpIX does strongly absorb red light. The present approach has several advantages over the prior art. First endogenous PpIX has a much shorter half-life in normal tissues (human and mouse, at least) than does HpIX, HpD or Photofrin^{®} II. This greatly reduces the danger of accidental phototoxic skin reactions in the days following treatment. Second, the ALA can be applied topically to certain types of lesions. This improves the specificity of the treatment, reduces the danger of accidental phototoxic reactions to a very low level, and greatly reduces the amount of both ALA and PpIX to which the entire body would be exposed if an equally effective dose of ALA were to be given systemically.

Both ALA and PpIX are normal products of metabolism, and are handled quite readily by the biochemical machinery of the body. However, since very large doses of ALA (like large doses of HpIX or HpD) are associated with a transient decrease in motor nerve conduction velocity, it is desirable to reduce the dose of ALA to the minimum that is still effective. Topical application requires much less ALA than systemic administration. Third, PpIX is rapidly inactivated by the photoactivating light. Following exposure of tissues containing PpIX to a therapeutic dose of photoactivating light, there is a substantial decrease in photosensitization of the tissues within the treatment volume. Consequently, if PpIX is induced by the topical application of ALA to specific lesions, the patient can be exposed to sunlight immediately post-treatment without danger of serious phototoxicity. Also, the dosimetry of the photoactivating light is great simplified. Fourth, ALA is an effective inducer of PpIX when given by mouth, by topical application, or by injection. In contrast, HpIX, HpD and Photofrin II are effective in most situations only when given by injection. The versatility of ALA enhances its acceptability for routine use by the medical profession, since the oral and topical routes of administration are much more convenient than the parenteral. Fifth, the normal and abnormal tissues that can be photosensitized by the administration of ALA are somewhat different from those that can be photosensitized by the administration of HpIX, HpD or Photofrin II. Consequently, ALA would be useful in clinical situations in which the other photosensitizers are not.

Thus the present technique is not merely another way to do what can be done already but is; in fact, a significant advance in therapeutic capability.

### EXAMPLE 1 ACNE

Acne is an inflammatory follicular papular and pustular eruption involving the skin. The treatment of acne using the method of the instant invention would be considered to be the treatment of either (a) endogenous lesions of the sebaceous apparatus of the skin due to intrafollicular hyperkeratosis or (b) exogenous bacteria cells present in the acne lesions, particularly *Propionibacterium (Corynebacterium) acne.*

Evaluation of PpIX induced fluorescence in 8 subjects with mild to moderate truncal acne was performed. Bacterial infections are frequently associated with lesions of acne, *e.g., P. acne.* Following evaluation of baseline acne lesion fluorescence, ALA solution 10 and 20% was applied to 10 5 cm² sites on the chest or back of volunteers and evaluated at times 0, 3, 8 and 24 hours after ALA application. One site of each concentration was also occluded with opaque film for 3 hours and evaluated at similar time points for comparison with unoccluded sites. Fluorescence of both acneiform lesions as well as surrounding normal skin was assessed visually using a 4 point grading system (0=none, 4=extremely severe) and documented photographically.

In all subjects, unoccluded sites had a gradual increase in PpIX fluorescence that was dose dependent, maximum at 8 hours, specific for acne lesions and spared normal surrounding skin. These sites had weak or no fluorescence by 24 hours. Little difference in fluorescence intensity was noted by lesion type (cornedones vs papules vs pustules) in the same subject, however, time to maximal fluorescence and maximal fluorescence intensity was variable from subject to subject. Lesions with surrounding erythema (larger papules and pustules) developed fluorescence extending to the clinical limit of erythema. Vehicle control sites remained at baseline. In contrast, occluded sites developed PpIX fluorescence in both acne lesions and normal surrounding skin that persisted longer than unoccluded sites and remained present at 24 hours.

## Claims

1. A precursor of PpIX in the biosynthetic pathway for heme that can induce synthesis and/or accumulation of PpIX or other endogenous porphyrins, their precursors and their photoproducts in non-malignant hyperproliferative lesions, for use in treating non-malignant hyperproliferative acne lesions of the skin, following exposure of said lesions to light having a wavelength within the photoactivating action spectrum of said PpIX to thereby induce photoactivation in said lesions, wherein the precursor is provided in a solution comprising 20% 5-aminolevulinic acid ("ALA").

2. A precursor of PpIX for use according to claim 1, wherein said treatment involves occlusion of said ALA-treated lesion for 3 hours.

## Patentansprüche

1. Ein Vorläufer von PpIX im Biosyntheseweg für Häm, der Synthese und/oder Akkumulation von PpIX oder anderen endogenen Porphyrinen, deren Vorläufern und deren Photoprodukten in nicht-malignen hyperproliferativen Läsionen induzieren kann, für den Einsatz in der Behandlung von nicht-malignen hyperproliferativen Akne-Läsionen der Haut, nach Exposition der Läsionen gegenüber Licht mit einer Wellenlänge innerhalb des photo-aktivierenden Aktionsspektrums des PpIX, um dabei Photo-Aktivierung in den Läsionen zu induzieren, wobei der Vorläufer in einer Lösung bereitgestellt wird, die 20 % 5-Aminolävulinsäure ("ALA") beinhaltet.

2. Ein Vorläufer von PpIX für den Einsatz gemäß Anspruch 1, wobei die Behandlung Okklusion der ALA-behandelten Läsionen für 3 Stunden involviert.

## Revendications

1. Précurseur de PpIX dans la voie de synthèse biologique de l'hème, pouvant induire la synthèse et/ou l'accumulation de PpIX ou d'autres porphyrines endogènes, de leurs précurseurs et photoproduits dans des lésions hyperprolifératives bénignes, à utiliser pour traiter des lésions acnéiques hyperprolifératives bénignes de la peau, par l'exposition ultérieure desdites lésions à de la lumière ayant une longueur d'onde comprise dans le spectre d'action photo-activante de ladite PpIX, afin d'induire une photo-activation dans lesdites lésions, dans lequel le précurseur est fourni dans une solution comprenant 20 % d'acide 5-aminolévulinique (« ALA »).

2. Précurseur de PpIX à utiliser selon la revendication 1, dans lequel ledit traitement nécessite l'occlusion desdites lésions traitées à l'ALA pendant 3 heures.
